(19) Europäisches Patentamt
European Patent Office
Office européen des brevets

(11) Veröffentlichungsnummer: **0 233 570**
**A2**

(12) # EUROPÄISCHE PATENTANMELDUNG

(21) Anmeldenummer: **87101740.6**

(22) Anmeldetag: **09.02.87**

(51) Int. Cl.[4]: **C12P 7/24**, C12N 1/20, //(C12N1/20,C12R1:06),(C12P7-/24,C12R1:06)

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 3597

(30) Priorität: **15.02.86 DE 3604874**

(43) Veröffentlichungstag der Anmeldung: **26.08.87 Patentblatt 87/35**

(84) Benannte Vertragsstaaten: **AT BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Cooper, Bryan Dr.**
**Waldparkstrasse 32**
**D-6800 Mannheim 1(DE)**

(54) **Verfahren zur Herstellung von Coniferylaldehyd und Mikroorganismus dafür.**

(57) Es werden neue Mikroorganismen beschrieben, mit deren Hilfe n-Eugenol in Coniferylaldehyd umgewandelt werden kann.

EP 0 233 570 A2

Xerox Copy Centre

## Verfahren zur Herstellung von Coniferylaldehyd und Mikroorganismus dafür

Die Erfindung betrifft ein Verfahren zur Herstellung von Coniferylaldehyd und dafür geeignete Mikroorganismen.

Coniferylaldehyd ist ein Zwischenprodukt für die Herstellung von Vanillin, einem Aromastoff, der in der Nahrungsmittelindustrie breite Verwendung findet. Der Aldehyd läßt sich leicht in Vanillin durch Erhitzen in Anwesenheit von Natronlauge umwandeln (Acta Chem. Scand. 3 , 86 (1949).

Es ist bereits angegeben worden, daß man mit Mikroorganismen n-Eugenol (A) über Ferulasäure (B) oxidativ abbauen kann (Agric. Biol. Chem. 41, 925 (1977). Hierbei entsteht neben Vanillin (C) stets in beträchtlicher Menge auch Vanillinsäure (D):

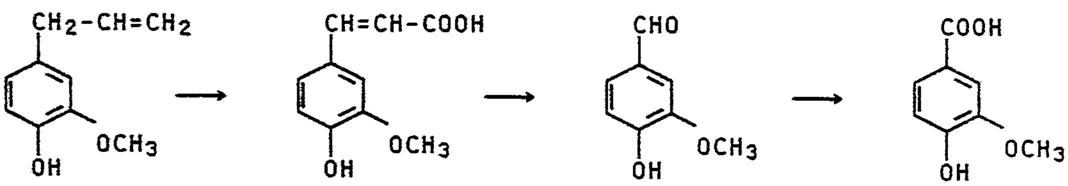

A B C D

Die Mikroorganismen für diese Reaktion sind jedoch nicht zugänglich, so daß das Verfahren nicht durchführbar ist.

In Agric. Biol. Chem. 47, 2639 (1983) ist weiter angegeben, daß man n-Eugenol über Coniferylaldehyd - (E)

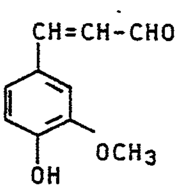

E

in B überführen kann. Dieses Verfahren ist ebenfalls nicht nacharbeitbar, da der verwendete Mikroorganismus nicht zur Verfügung steht.

Es wurden nun Mikroorganismen gefunden, die in der Lage sind, n-Eugenol in Coniferylaldehyd umzuwandeln.

Gegenstand der Erfindung ist das Bacterium Arthrobacter globiformis DSM 3597 und dessen n-Eugenol in Coniferylaldehyd umwandelnde Mutanten.

Arthrobacter globiformis DSM 3597 läßt sich wie folgt taxonomisch beschreiben:

1. Gestalt der Zellen (Flüssigkultur in Nutrient Broth bei 28°C und 250 UpM beobachtet):

Stäbchen mit Abmessungen 0,5 -0,8 × 1 -5 µm. Im Verlaufe des Wachstums treten unregelmäßige Formen auf, die wesentlich größer sein können. Gegen Ende des Wachstums liegen vorwiegend kokkoide Formen vor (0,5 -1,0 µm Durchmesser), die vorzugsweise in großen Verbänden zusammenhängen.

2. Beweglichkeit: nicht beweglich.
3. Sporen: keine beobachtet.
4. Gram-Färbung: positiv in allen Wachstumsphasen.
5. Wachstum auf Minimalmedium + 1 % Glycerin: positiv in 3 bis 6 Tagen.

Zusammensetzung des Minimalmediums:

0,5 % Ammoniumsulfat, 0,15 % Kaliumdihydrogenphosphat, 0,36 % Dikaliumhydrogenphosphat, 0,05 % Magnesiumsulfat-7-hydrat, 0,005 % Mangansulfat-1-hydrat, 0,2 % Spurenelementlösung (= "SL"), 1,8 % Agar.

Zusammensetzung der SL:

200 mg/l Eisen(II)sulfat-monohydrat, 10 mg/l Zink(II)sulfat-4-hydrat, 3 mg/l Mangan(II)sulfat-4-hydrat, 30 mg/l Borsäure, 20 mg/l Cobalt(II)chlorid-6-hydrat, 1 mg/l Kupfer(II)chlorid-2-hydrat, 2 mg/l Nickel(II)chlorid-6-hydrat, 3 mg/l Natriummolybdat-2-hydrat, 500 mg/l EDTA in destilliertem Wasser.

6. Vitaminbedürfnis: nicht vorhanden.

Stimulierung des Wachstums auf Glycerin-Minimalmedium durch Zugabe von 0,1 mg/l Biotin: stark ausgeprägt.

7. Koloniemorphologie: flache, glänzende, vorwiegend hellgelbe Kolonien. Weiße Varianten treten spontan auf.

8. Wachstum auf Kälberserum-Pepton-Tellurit-Agar (37°C): kleine, schwarze Kolonien innerhalb von 12 Tagen.

9. Anaerobes Wachstum: kein Wachstum innerhalb von acht Tagen mit 1 % D-Glucose als Kohlenstoffquelle im Nutrient Broth-Medium.

10. Verwertbare Stickstoffquellen: Ammoniumsulfat, Kaliumnitrat, Harnstoff sowie nach einer Eingewöhnungsphase elementarer Stickstoff.

11. Temperaturbereich: Wachstum bei 22°C und 37°C.

Optimum: 28°C.

12. C-Quellen: Wachstum auf folgenden C-Quellen (1 g/l), getestet im Minimalmedium, angereichert mit 0,1 mg/l Biotin.

Glycin: positiv
Tyramin: positiv
Harnsäure: positiv
4-Hydroxibenzoesäure: positiv
3,4-Dihydroxibenzoesäure: positiv
3-Hydroxibenzoesäure: positiv
D-Xylose: positiv
Melezitose: positiv
D-Glucuronsäure: positiv
D-Glucosamin: positiv
myo-Inosit: positiv
2-Oxoglutarsäure: positiv

13. Katalase: positiv

Geeignete Mutanten von Arthrobacter globiformis DSM 3597 erhält man durch spontane oder induzierte Mutation. Beispielsweise kann die mutagene Behandlung durch Bestrahlung mit UV-Strahlen oder durch Behandlung mit mutagenen Substanzen wie N-Methyl-N'-nitro-N-nitrosoguanidin (MNNG) durchgeführt werden. Anschließend werden die so erhaltenen Mutanten nach ihrer Fähigkeit selektiert, n-Eugenol in Coniferylaldehyd umzuwandeln, ohne den Coniferylaldehyd weiter zu verändern.

Gegenstand der Erfindung ist weiter ein Verfahren zur Herstellung von Coniferylaldehyd, welches darin besteht, daß man geeignete Mutanten des Stammes Arthrobacter globiformis DSM 3597 in Gegenwart von n-Eugenol kultiviert.

Zur Durchführung des erfindungsgemäßen Verfahrens werden geeignete Mutanten des Stammes Arthrobacter globiformis DSM 3597 auf ein n-Eugenol enthaltendes Nährmedium überimpft und darin vorzugsweise bei pH 6-8 und 22-37°C inkubiert. Die Fermentation kann kontinuierlich oder diskontinuierlich erfolgen.

Die Zellen des verwendeten Stammes, die auch in Form von ruhenden, nicht wachsenden Zellen verwendet werden können, läßt man direkt auf das Substrat einwirken. Es können beliebige, bekannte Inkubationsverfahren angewendet werden, besonders bevorzugt sind aber Fermenter in Form von tiefen, belüfteten und gerührten Tanks. Sehr gute Ergebnisse werden durch Inkubation eines flüssigen Nährmediums erhalten.

Die Wahl des Nährmediums zur Züchtung des Mikroorganismus ist nicht kritisch. Besonders geeignet sind Nährmedien, die Kohlenstoffquellen, Stickstoffquellen, anorganische Salze und gegebenenfalls geringe Mengen an Spurenelementen und Vitaminen enthalten. Als Stickstoffquellen können anorganische oder organische Stickstoffverbindungen oder Materialien, die diese Verbindungen enthalten, verwendet werden. Beispiele sind: Ammoniumsalze, Nitrate, Maisquellwasser, Bierhefeautolysat, Sojabohnenmehl, Weizengluten, Hefeextrakt, Hefe, Harnstoff und Kartoffelprotein. Als Kohlenstoffquellen können beispielsweise neben dem als Ausgangsmaterial genannten n-Eugenol Zucker, wie D-Glucose, Polyole, wie Glycerin, oder andere Alkohole, wie Ethanol, aber auch jede der in der Stammbeschreibung genannten Kohlenstoffquellen verwendet werden.

Beispiele für anorganische Salze sind die Salze von Calcium, Magnesium, Mangan, Kalium, Zink, Kupfer, Eisen und anderen Metallen. Als Anion der Salze ist besonders das Phosphation zu nennen. Gegebenenfalls werden dem Nährmedium Wachstumsfaktoren zugesetzt, wie zum Beispiel Biotin. Das Mischungsverhältnis der genannten Nährstoffe hängt von der Art der Fermentation ab und wird im Einzelfall festgelegt.

Im allgemeinen eignen sich zur Durchführung des erfindungsgemäßen Verfahrens n-Eugenolkonzentrationen von etwa 0,5 bis 100 g/l, bevorzugt sind Konzentrationen von etwa 0,5 bis 50 g/l.

Die Züchtungsbedingungen werden so festgelegt, daß die bestmöglichen Ausbeuten erreicht werden. Bevorzugte Züchtungstemperaturen liegen bei 24 bis 32°C. Vorzugsweise wird der pH-Wert in einem Bereich von 5-9, vorzugsweise 6-8 festgehalten. Im allgemeinen ist eine Inkubationsdauer von 15-100 Stunden ausreichend. Innerhalb dieser Zeit reichert sich die maximale Menge des gewünschten Produkts im Medium an.

Die erforderliche Menge n-Eugenol kann dem Nährmedium am Anfang auf einmal oder während der Züchtung in mehreren Portionen zugegeben werden.

Abgesehen von der vorbeschriebenen Inkubation des erfindungsgemäßen Mikroorganismus können nach dem erfindungsgemäßen Verfahren auch die Zellen des Mikroorganismus, d.h. ruhende, nichtwachsende Zellen, in di rekten Kontakt mit dem als Ausgangsmaterial genannten Substrat n-Eugenol gebracht werden. In diesem Falle werden die physikalischen Bedingungen, wie z.B. Temperatur, pH-Wert, Belüftung usw., ähnlich wie oben angegeben gewählt.

Die Verwendung von Aktivkohle oder Divinylbenzyl-quervernetztem Polystyrol als Adsorbens für das eingesetzte n-Eugenol ist vorteilhaft, weil diese die Mikroorganismen vor der baktericiden Wirkung des n-Eugenols schützen. So kann das n-Eugenol in höherer Konzentration eingesetzt werden.

Das gebildete und ins Medium ausgeschiedene Produkt Coniferylaldehyd kann nach bekannten Verfahren abgetrennt und gereinigt werden. Zur Abtrennung des Produkts können die entstandenen Kristalle durch Zentrifugation oder Filtration abgetrennt werden. Diese Rohkristalle können durch Umkristallisation oder durch Extraktion mit einem üblichen Lösungsmittel, wie Methyl-tert.-butylether, gereinigt werden. Der Extrakt kann getrocknet und der Coniferylaldehyd durch Umkristallisation gereinigt werden. Alternativ hierzu kann der Coniferylaldehyd durch Chromatographie an einer üblichen Chromatographiesäule, wie einer Silicagel-Säule, von nicht erwünschten Nebenprodukten abgetrennt werden.

Die Beispiele erläutern die Erfindung.

Stammisolierung

Beispiel 1

Ein Agarmedium wurde hergestellt, welches folgende Komponenten enthielt:

n-Eugenol 0,5 g/l
®Amberlite XAD-2 (= Divinyl-benzyl-quervernetztes Polystyrol) 5,0 g/l
Ammoniumsulfat 0,5 g/l
Kaliumdihydrogenphosphat 1,5 g/l
Dikaliumhydrogenphosphat 3,6 g/l
Magnesiumsulfat-7-hydrat 0,5 g/l
Mangansulfat-1-hydrat 0,05 g/l
Biotion 0,1 mg/1
SL 2,0 mg/1
Agar 18,0 g/l

Die Sterilisation erfolgte bei 121°C für 15 min. Die beiden Phosphatsalze wurden getrennt vom restlichen Medium hitzesterilisiert. Das n-Eugenol wurde nicht sterilisiert und bei einer Mediumtemperatur von 50°C dem Medium hinzugefügt und gut eingerührt. Agarplatten mit einem Volumen von 40 ml/Platte wurden gegossen und nach dem Erstarren mit je 0,1 ml verschiedener Suspensionen von Humus-haltigen Bodenproben bechickt. Diese Platten wurden 14 Tage bei 25°C bebrütet. Hellgelbe Kolonien wurden abgeimpft und in der üblichen Weise gereinigt.

Diese Stämme wurden dann einzeln in 10 ml eines sterilen Nährmediums (im folgenden als HE-Medium bezeichnet) eingeimpft, welches 0,5 % Hefeextrakt, 0,5 % Ammoniumsulfat, 0,15 % Kaliumdihydrogenphosphat, 0,36 % Dikaliumhydrogenphosphat, 0,05 % Magnesiumsulfat-7-hydrat, 0,1 mg/l Biotin, 0,2 % (v/v) SL und 0,005 % Mangansulfat-1-hydrat enthielt.

Die Ansätze wurden 16 h bei 25°C und 250 UpM inkubiert. Nach Ablauf dieser Zeit wurden sie mit 10 ml des gleichen Mediums versetzt, welches jedoch 0,4 g/l n-Eugenol enthielt. Das n-Eugenol wurde als 50%ige Lösung in N,N-Dimethylformamid (DMF) zugegeben. Nach weiteren 24 h Inkubation wurden die Ansätze mit einem Volumen Essigsäureethylester extrahiert. Je 5 µl dieser Extrakte wurden einzeln auf handelsübliche Kieselgelplatten aufgetragen und mit einem Laufmittelsystem bestehend aus n-Butanol, 25 % Ammoniak, 96%igem Ethanol und Wasser im Verhältnis 120 : 60 : 30 : 15 entwickelt. Nach der Entwicklung wurden das n-Eugenol und seine Abbauprodukte in einer Iodkammer sichtbar gemacht. Stämme, die das n-Eugenol vollständig umsetzten und die oben beschriebenen physiologischen und morphologischen Eigenschaften aufwiesen, gehören zur Art Arhrobacter globiformis. Wie bereits erwähnt, ist ein solcher Stamm hinterlegt.

Isolierung von geeigneten Mutanten

Beispiel 2

Ein 100 ml Kolben wurde mit 20 ml eines sterilen Flüssignährmediums beschickt, wie es in Beispiel 1 beschrieben worden ist. Dieser Ansatz wurde mit einer Öse DSM 3597 von einer Agarplatte gleicher Zusammensetzung beimpft und über Nacht bei 25°C und 250 UpM schüttelnd inkubiert.

Ein zweiter Kolben gleicher Zusammensetzung, jedoch mit Zusatz von 0,08 ml einer 50%igen n-Eugenollösung (in DMF), wurde mit 4 ml dieser Vorkultur beimpft und ebenfalls bei 25°C und 250 UpM unter Schütteln inkubiert. Nach 4,5 h wurde der Umsatz des zugesetzten n-Eugenols mittels Dünnschichtchromatographie, wie sie in Beispiel 1 beschrieben wurde, überprüft. Erfahrungsgemäß beträgt der Umsatz zu diesem Zeitpunkt etwa 50 %. Nach 5 h wurde dann 1,0 ml einer 5%igen MNNG-Lösung (in DMF) hinzugegeben. Die Kultur wurde dann 10 min weitergeschüttelt. Danach wurde die gesamte Kultur unter Einhaltung steriler Bedingungen bei 4°C 10 min abzentrifugiert und der Überstand verworfen. Die abzentrifugierten Zellen wurden in 10 ml einer wäßrigen Lösung, die 10 % Glycerin und 5 % Lactose enthält, aufgenommen. Diese Suspension wurde bis zum Gebrauch bei -85°C eingefroren. Der Lebendzelltiter dieser Suspension wurde in der üblichen Weise bestimmt. Eine Suspension der aufgetauten Zellen wurde dann hergestellt, die 100 lebendfähige Zellen pro 0,1 ml enthält. Je 0,1 ml dieser Suspension wurden dann auf 100 Glycerin-Minimalmedium-Platten ausplattiert und 4 Tage bei 25°C bebrütet. Diese wurden in der üblichen Weise mit Samtstempeln auf ein n-Eugenol-haltiges Nähragar überstempelt, wie es in Beispiel 1 beschrieben wurde. Diese Platten wurden 2 Tage bei 25°C bebrütet. Kolonien, die auf dem Glycerin-Minimalmedium gutes, auf dem n-Eugenol-Medium schlechtes oder gar kein Wachstum zeigten, wurden isoliert. Diese Klone wurden dann in Flüssigkultur geprüft, wie dies in Beispiel 1 beschrieben wurde. Ansätze, die nach insgesamt 40 h Inkubationszeit eine leuchtend gelbe Farbe aufwiesen, wurden dünnschichtchromatographisch untersucht. Hierzu wurde authentischer Coniferylaldehyd als Referenz herangezogen. Klone, die das eingesetzte n-Eugenol unter diesen Bedingungen ausschließlich zu Coniferylaldehyd umsetzten, wurden isoliert und in der üblichen Weise konserviert.

Umsetzung von n-Eugenol

Beispiel 3

Ein 1 Liter-Fermenter wurde mit 900 ml eines sterilen Mediums beschickt, welches folgende Bestandteile enthielt: 2,0 g Glycerin, 1,0 g Ammoniumsulfat, 1,5 g Kaliumdihydrogenphosphat, 3,6 g Dikaliumhydrogenphosphat, 0,5 g Magnesiumsulfat-7-hydrat, 0,05 g Mangansulfat-1-hydrat, 2 ml SL und 0,1 mg Biotin.

Eine 100 ml Vorkultur bestehend aus dem gleichen Medium wurde mit einer Mutante von Arthrobacter globiformis (vgl. Beispiel 2) 16 h bei 25°C und 250 UpM bebrütet. Der 1 Liter-Fermenter wurde mit dieser Vorkultur beimpft und bei 25°C, 500 UpM und 0,2 Volumenteilen Luft pro Volumenteil Fermentationslösung und pro min Luft betrieben. 4 h nach dem Beimpfen wurde 0,4 ml n-Eugenol (50 % in Dimethylformamid) hinzugegeben. Der Abbau des n-Eugenols wurde dünnschichtchromatographisch verfolgt, vgl. Beispiel 1. Nach weiteren 2 h und 6 h wurden weitere 0,4 ml n-Eugenollösung hinzugegeben.

Die Fermentation wurde abgebrochen, nachdem das n-Eugenol umgesetzt war. Der gesamte Ansatz wurde mit Methyl-tert.-butylether dreimal extrahiert. Die Extrakte wurden getrocknet und der Rückstand in 50 ml Acetonitril aufgenommen. Die Konzentration des gebildeten Coniferylaldehyds wurde in der üblichen Weise mittels HPLC bestimmt. Sie betrug 185 mg/l, bezogen auf das ursprüngliche Fermentationsmedium, was einer Ausbeute von 44 %, bezogen auf das eingesetzte n-Eugenol, entspricht.

Beispiel 4

In einem Fermenter wurden 800 ml eines sterilen Nährmediums hergestellt, das folgende Bestandteile enthielt: 5 g Hefeextrakt, 5 g Ammoniumsulfat, 0,5 g Magnesiumsulfat-7-hydrat, 0,05 g Mangansulfat-1-hydrat, 0,1 mg Biotin, 1,5 g Kaliumdihydrogenphosphat, 3,6 g Dikaliumhydrogenphosphat, 2 ml SL und 20 g Aktivkohlepulver.

Eine 200 ml Vorkultur einer gemäß Beispiel 2 erhaltenen Mutante wurde in dem gleichen Medium hergestellt, welches jedoch keine Aktivkohle enthielt. Die Beimpfung erfolgte mit einer Öse Zellen. Die Inkubation erfolgte bei 25°C und 250 UpM schüttelnd.

Vor Beimpfung des Fermenters wurden 2 g n-Eugenol hinzugegeben und 10 min eingerührt. Der Fermenter wurde mit der Vorkultur beimpft. Die Inkubation erfolgte bei 25°C, 500 UpM und einer Belüftungsrate von 0,2 vvm Luft für 28 h. Der Umsatz wurde in der üblichen Weise mittels Dünnschichtchromatographie geprüft. Nachdem das n-Eugenol vollständig umgesetzt worden war, wurde eine 50 ml Probe so lange mit Methylenchlorid extrahiert, bis die wäßrige, Aktivkohle-haltige Phase kein Coniferylaldehyd mehr enthielt. Die vereinigten Extrakte wurden bis zur Trockenheit eingedampft. Der Rückstand wurde in 50 ml Wasser gelöst und mittels HPLC analysiert. Die Ausbeute an Coniferylaldehyd betrug 1,25 g/l, bezogen auf die ursprüngliche Fermentationsbrühe (65 %).

Beispiel 5

Die Umsetzung erfolgte analog dem Beispiel 4. Anstelle der Aktivkohle wurden jedoch nach 8 h Fermentationsdauer 20 g ®Amberlite XAD-2 + 2 g n-Eugenol (in 100 ml dest. Wasser) dem Fermentationsmedium beigefügt. Die Ausbeute betrug 750 mg/l (40 %).

Isolierung des Coniferylaldehyds

Beispiel 6

Die gemäß Beispielen 3 -5 erhaltenen Fermentationsbrühen wurden mit Methyl-tert.-butylether so lange ausgeschüttelt, bis kein Coniferylaldehyd in der wäßrigen Phase mehr nachgewiesen werden konnte. Die vereinigten Extrakte wurden bei 40°C im Rotationsverdampfer eingedampft.

Der Rückstand wurde in 100 ml Methyl-tert.-butylether gelöst und klar filtriert. Das Filtrat wurde mit 200 ml n-Heptan versetzt und im Rotationsverdampfer bei 40°C auf ein Volumen von 150 ml eingeengt. Der Coniferylaldehyd kristallisierte unter diesen Bedingungen aus. Er wurde zweimal aus siedendem n-Hexan umkristallisiert. Es wurden hellgelbe Nadeln (Fp. 84°C) erhalten.

## Ansprüche

1. Arthrobacter globiformis DSM 3597 und dessen n-Eugenol in Coniferylaldheyd umwandelnde Mutanten.

2. Verfahren zur Herstellung von Coniferylaldehyd, dadurch gekennzeichnet, daß man Mutanten von Arthrobacter globiformis DSM 3597 gemäß Anspruch 1 in Gegenwart von n-Eugenol kultiviert.